# EUROPEAN PATENT APPLICATION

(11) **EP 4 410 763 A1**
(43) Date of publication of application: **07.08.2024**
(21) Application number: 23155185.4
(22) Date of filing: 06.02.2023
(51) Int. Cl.: C07C 1/30, C07C 11/06, C07C 11/04

(54) **PROCESS FOR CATALYTIC DEHYDROBROMINATION OF ONE OR MORE C2-10-BROMOHYDROCARBONS TO THE CORRESPONDING C2-10-ALKENES**

(71) Applicant: Sulzer Management AG, 8401 Winterthur (CH)
(72) Inventor: The designation of the inventor has not yet been filed
(74) Representative: Henkel & Partner mbB

(57) **Abstract**

The present invention relates to a process for catalytically reacting a composition containing one or more C₂₋₁₀-bromohydrocarbons to the corresponding C₂₋₁₀-alkenes, wherein the reaction is performed in the presence of a catalyst comprising a support material and thereon at least 200 ppm of at least one metal being selected from the group consisting of transition metals, noble metals and arbitrary combinations of two or more thereof.

## Description

The present invention relates to a process for catalytic dehydrobromination or reacting, respectively, a composition containing one or more C₂₋₁₀-bromohydro-carbons to the corresponding C₂₋₁₀-alkenes.

Olefins or alkenes, respectively, and particularly C₂₋₁₀-alkenes, such as ethylene and propylene, are valuable raw material for chemical syntheses and belong in fact to the most important starting materials in the petrochemical industry. For instance, ethylene is the starting material for producing polyethylene and propylene is the starting material for producing polypropylene, which are two of the most widely used polymers, since they are odorless, skin-friendly, physiologically harmless and have excellent mechanical properties. For example, polyethylene and polypropylene are used for packings, films, textiles, caps, medical products, mattress ticks and many other articles. Furthermore, ethylene and propylene are used for the production of other important chemicals, such as for producing ethylene glycol, propylene glycol, acrylonitrile, acrylic acid and propylene oxide.

C₂₋₁₀-alkenes are commonly produced by a direct catalytic dehydrogenation of the corresponding C₂₋₁₀-alkane, such as propylene by a direct catalytic dehydrogenation of propane. The catalytic dehydrogenation of propane to propylene is usually performed at a relatively high temperature of 550 to 680°C and at a comparable low-pressure, which is typically below atmospheric pressure. However, such processes based on the direct catalytic dehydrogenation of C₂₋₁₀-alkanes are - among others on account of the required high reaction temperature - energy intensive and ecologically harmful, if non-regenerable fuels, such as coal or the like, forming tremendous amounts of carbon dioxide during their combustion, are used to generate the required energy for maintaining the required high reaction temperature. The predominantly used direct dehydrogenation processes utilize circulating catalysts or multiple reactors circulating in sequence production-purge-regeneration-purge. This requirement derives from the alkane-alkene equilibrium limitation, which is mitigated by the high temperature and/or low-pressure operation. All in all, these processes based on the direct catalytic dehydrogenation of a C₂₋₁₀-alkane have the drawback of high capital expenditures, of high operational costs and of producing high amounts of carbon dioxide.

Other processes for producing C₂₋₁₀-alkenes are known, such as for instance processes based on a chemical looping. For example, a process for producing propylene is known, in which a bromopropane is reacted with copper oxide to form propylene, copper bromide and water, before the copper oxide is regenerated by reacting copper bromide with oxygen to bromine and copper oxide. However, this process is very energy intensive.

Also known are processes, in which propane is brominated with bromine and the so obtained bromopropane is dehydrobrominated to propylene and hydrogen bromide, before bromine is recovered from the hydrogen bromide by oxidizing hydrogen bromide to bromine and water. However, the thermal dehydrobromination of C₂₋₁₀-bromoalkanes to the corresponding C₂₋₁₀-alkenes is slow and thus not economical, because it requires too long residence times and high temperatures in order to achieve a suitable reaction.

In view of this, the object underlying the present invention is to provide a process for reacting a C₂₋₁₀-bromohydrocarbon to the corresponding C₂₋₁₀-alkene, which can be operated in an industrial scale, has a comparable low energy demand, is ecologically friendly and in particular does not produce high amounts of carbon dioxide and does not require high capital expenditures.

In accordance with the present invention, this object is solved by providing a process for catalytically reacting a composition containing one or more C₂₋₁₀-bromohydrocarbons to the corresponding C₂₋₁₀-alkenes, wherein the reaction is performed in the presence of a catalyst comprising a support material and thereon at least 200 ppm of at least one metal being selected from the group consisting of transition metals, noble metals and arbitrary combinations of two or more thereof.

This solution bases on the finding that a catalyst comprising a support material and thereon at least 200 ppm of at least one metal being selected from the group consisting of transition metals, noble metals and arbitrary combinations of two or more thereof particularly efficiently catalyzes the dehydrobromination of C₂₋₁₀-bromohydrocarbons, such as of a C₂₋₁₀-bromoalkane to the corresponding C₂₋₁₀-alkene and hydrogen bromide or of a C₄₋₁₀-dibromoalkane to the corresponding C₄₋₁₀-dialkene and hydrogen bromide or of a C₄₋₁₀-mono-bromoalkene to the corresponding C₄₋₁₀-dialkene and hydrogen bromide. A further advantage of the aforementioned catalyst is that it is very stable. Without wishing to be bound by any theory, it is considered that that a catalyst comprising a support material and thereon at least 200 ppm of at least one metal being selected from the group consisting of transition metals, noble metals and arbitrary combinations of two or more thereof is modified during the time in which it is subjected in a contact with a bromo-containing hydrocarbon to supported metal bifunctional catalyst comprising dehydrogenation and acid functionalities. The promotion with bromine creates acid sites, which are localized directly on the surface of the support material and are very efficient in catalyzing a dehydrobromination reaction. Moreover, an intimacy between the transition metal(s) and/or noble metal(s) and the acid functions in this bifunctional catalyst generates unique catalytic properties for catalytic dehydrobromination making them very active and stable in the reaction. It has been further found in the present invention that the catalyst being used in accordance with the present invention does not convert - even after being modified to the metallic bifunctional catalyst in the presence of a bromo-containing hydrocarbon - the produced C₂₋₁₀-alkene to any secondary product, such as an oligomer or an aromatic. It was further shown in the present invention that this catalyst shows a very high activity, a very high selectivity and a very stable performance both, in the presence of hydrogen as well as in the absence of hydrogen.

In accordance with the present invention, a composition containing one or more C₂₋₁₀-bromohydrocarbons is catalytically reacted to the corresponding C₂₋₁₀-alkenes in the presence of the catalyst. Corresponding C₂₋₁₀-alkene means any C₂₋₁₀-alkene, which has the same number of carbon atoms as the C₂₋₁₀-bromohydrocarbon, from which it is obtained during the reaction. In case of C₄₋₁₀-alkenes, the C₄₋₁₀-alkene may be a monoalkene, a dialkene or a mixture of monoalkene and dialkene. The term "C₂₋₁₀-bromohydro-carbon" denotes C₂₋₁₀-monobromohydrocarbons, C₂₋₁₀-dibromohydrocarbons and higher C₂₋₁₀-bromohydrocarbons. Analogously, "C₂₋₁₀-bromoalkane" denotes C₂₋₁₀-monobromoalkanes, C₂₋₁₀-dibromoalkanes and higher C₂₋₁₀-bromoalkanes and "C₂₋₁₀-bromoalkene" denotes C₂₋₁₀-monobromoalkenes, C₂₋₁₀-dibromoalkenes and higher C₂₋₁₀-bromoalkenes.

In accordance with the present invention, the catalyst used in the process comprises a support material. Preferably the catalyst used in the process comprises at least 75% by weight, more preferably least 75% by weight to 99.6% by weight, still more preferably least 85% by weight to 99.6% by weight and most preferably least 95% by weight to 99.6% by weight of support material.

In a further development of the idea of the present invention, it is suggested that the support material of the catalyst used in the process has a specific surface area, measured by nitrogen adsorption, of at least 5 m²/g more, preferably of at least 20 m²/g and more preferably of at least 50 m²/g.

The present invention is not particularly limited concerning the chemical nature of the support material. Good results are, however, in particular obtained, when the support material is selected from the group consisting of aluminas, silicas, silica gels, silica-aluminas, zeolites, zirconias, titanias, niobias, silicon carbides, carbons, aluminophosphates molecular sieves, metalloaluminophosphate molecular sieves, amorphous aluminophosphates and arbitrary combinations of two or more thereof.

In accordance with a particularly preferred embodiment of the present invention, the support material of the catalyst is gamma-alumina oxide and/or boehmite.

Alumina of a high purity of 99.99% or even higher is preferably be synthesized by the alkoxide route. Alkoxide route is known production technology, which is based on hydrolysis of aluminium alkoxide. In this process, the alcohol(s) of the aluminium alkoxide is/are recycled back to the synthesis. The alumina is produced either as co-product with synthetic linear alcohols (Ziegler method) or directly from aluminium metal (on-purpose route). The Ziegler route for the synthesis of alumina involves the oligomerization of ethylene using triethylaluminium followed by oxidation. The triethylaluminium may be produced by reacting aluminium, ethylene and hydrogen gas. In the production process, two-thirds of the triethylaluminium produced is recycled back into the reactor and only one-third is used to produce the alcohol. The recycling step is used to produce triethylaluminium at a higher yield and in less time. Triethylaluminium reacts with ethylene to form higher molecular weight trialkylaluminium. The number of equivalents of ethylene n equals the total number of monomer units being grown on the initial ethylene chains, wherein n = x + y + z, wherein x, y, and z are the number of ethylene units per chain. Trialkylaluminium is oxidized with air to form aluminium alkoxides, and finally hydrolyzed to aluminium hydroxide and the desired alcohols.
1.

   Al+3 C₂H₄ +1.5 H₂→ Al(C₂H₅)₃
2.

   Al(C₂H₅)₃ + n ethylene → Al((CH₂CH₂)ₙCH₂CH₃)₃
3.

   Al((CH₂CH₂)ₙCH₂CH₃)₃+ O₂ → Al(O(CH₂CH₂)ₙCH₂CH₃)₃
4.

   Al(O(CH₂CH₂)ₙCH₂CH₃)₃ → Al(OH)₃ + CH₃CH₂(CH₂C₂)ₘOH

In accordance with another embodiment of the present invention, the alumina used as support material is selected from the group consisting of aluminium oxyhydroxide, gamma-alumina, boehmite, diaspore, transitional aluminas, ρ-alumina and arbitrary combinations of two or more thereof. Suitable examples for transitional aluminas are α-alumina, β-alumina, γ-alumina, δ-alumina, ε-alumina and κ-alumina, whereas suitable examples for ρ-alumina are aluminium trihydroxide, such as gibbsite, bayerite, nordstrandite and doyelite.

In accordance with an alternative embodiment of the present invention, the support material of the catalyst used according to the present invention is a silica. Suitable examples for sources of silica are silicates, precipitated silicas, for example, Zeosil range available from Rhodia, fumed silicas, for example, Aerosil-200 available from Degussa Inc., New York, N.Y., silicon compounds, such as tetraalkyl orthosilicates, for example, tetramethyl orthosilicate (TMOS) and tetraethylorthosilicate (TEOS), colloidal silicas or aqueous suspensions thereof, for example Ludox-HS-40 sol available from E.I. du Pont de Nemours, Wilmington, Del., silicic acid, alkali-metal silicate, or any combination thereof. Other suitable examples of amorphous silica include silica powders, such as Ultrasil VN3SP (commercially available from Degussa), which has the advantages of being very cheap and of being easily available in large commercial scale quantities. Other examples of suitable solid silica sources are granulated hydrophilic fumed silicas, for example AEROPERL^{®} 300/30, mesoporous silica grade EXP & high surface area precipitated silica SIPERNAT from Evonik, HiSil 233 EP (available from PPG Industries) and Tokusil (available from Tokuyama Asia Pacific). In addition, suitable amorphous silica sources include silica sols, which are stable colloidal dispersions of amorphous silica particles in an aqueous or organic liquid medium, preferably water.

Non-limiting examples of suitable silica sols include those sold under the tradenames Nyacol (available from Nyacol Nano Technologies, Inc. or PQ Corp.), Nalco (available from Nalco Chemical Company), Ultra-Sol (available from RESI Inc), Ludox (available from W.R. Grace Davison), NexSil (available from NNTI). Many silica sols are prepared from sodium silicate and inevitably contain sodium. It is, however, been found in the present invention that the presence of sodium ions can cause sintering of the silica body at high temperature and/or affect catalytic performance. Therefore, if silica sols containing sodium are used, the silica sol is preferably subjected to at least one step of ion exchange in order to reduce its sodium content or even completely remove sodium therefrom. In order to avoid the purification of the silica sol with one or more ion exchange steps, it is preferred to use a silica sol containing very little or, ideally, no detectable traces of sodium and have a pH value of less than 7. Most preferably, the silica sol used for the preparation of the catalyst is slightly acidic with or without addition of one or more polymeric stabilizers. Non limiting examples of silica sols that contain no detectable traces of sodium are Bindzil 2034DI, Levasil 200, Nalco 1034A, Ultra-Sol 7H or NexSil 20A.

In a further development of the idea of the present invention, it is proposed to use silica dispersion prepared with alkylammonium for the preparation of the catalyst. Non-limiting examples of commercially low sodium silica sols stabilized by ammonia or alkylammonium cations include LUDOX TMA (available from W.R. Grace Davison) or VP WR 8520 from Evonik.

Silica sols with higher silica content than 30% by weight and even up to 50% by weight are for example W1250, W1836, WK341, WK7330 from Evonik.

Silica sols used to form a support are commercially also available as aquasols or organosols containing dispersed colloidal silica particles. For example, sodium silicate can be used as a silica sol. Otherwise, a silica gel, fumed or pyrogenic silica may also be used to provide a silica binder in the molecular sieve catalyst. Silicic acid is another possible source of silica. Advantageously, the binder contains low amount of sodium below 1000 ppm.

In accordance with a particular embodiment of the present invention, a silica sol or a combination of silica sol with precipitated or fumed silica is used as source of silicon for preparing the catalyst.

Also one or more zeolites may be used as support material of the catalyst to be used in the process of the present invention. A preferred zeolite is a so-called "medium pore size zeolite", which is a zeolite having a pore size of 0.5 to 0.65 nm measured by argon adsorption, meaning a zeolite, which has a pore size between the pore size of a "small pore size zeolite", such as an A-type zeolite, and the pore size of a "large pore size zeolite", such as a mordenite, an X-type zeolite and a Y-type zeolite), i.e. a Zeolite containing 10-membered rings in the crystal framework thereof. Examples of medium pore size zeolites are ZSM 5, ZSM-8, ZSM-11, ZSM-12, ZSM-21, ZSM-23, ZSM-35 and ZSM-38, most preferred are ZSM-5, ZSM-8, ZSM-11, ZSM-35 (Ferrierite) and ZSM-38. The Zeolite has preferably an Si/AI atomic ratio of from 11 to 5000, more preferably from 50 to 4000 and most preferably from 150 to 2000 as measured by elemental analysis.

The zeolite may contain 0.01 to 10 wt% of phosphorus. Such a modification is for example carried out by treating a zeolite with a phosphorous compound in an aqueous or a non-aqueous medium, by chemical vapor deposition of an organic phosphorous compound or by impregnation. The catalyst may be pre-formulated with binder or not. A preferred phosphorous compound is phosphoric acid, NH₄H₂PO₄ or (NH₄)₂HPO₄.

The zeolite may be exchanged with Ca-, Mg-, La- and/or Ce-compounds or directly with the salts of the transition and/or noble metal(s) being provided on the support material of the catalyst to be used according to the present invention. The aforementioned compounds bay be selected from organic compounds, salts, hydroxides and oxides. These compounds may also contain phosphorus. It is preferred that these compounds are present in solubilized form, before they are brough into contact with the zeolite or by forming a solution when being in contact with the zeolite.

The zeolite - before modification with a metal and phosphorous - may be calcined, steamed, ion-exchanged, treated with acid solution or it may have been subjected to another treatment leading to dealumination. Dealumination of the zeolite can be performed simultaneously with the phosphorous modification.

In order to further improve the resistance of the zeolite to coking deactivation, it is suggested in a further development of the idea of the present invention that - prior to the contact with the hydrocarbon feedstock - the above-mentioned Zeolite is subjected to a heat treatment at 500°C or more in the presence of steam. Preferably, this heat (steaming) treatment is conducted at 500°C to 900°C under a steam partial pressure of 0.01 MPa or more.

In accordance with an alternative embodiment of the present invention, a carbon is used as source of silicon for preparing the catalyst used in the process of the present invention.

Preferably, the carbon is selected from graphite, carbon black, coke, petroleum coke and any arbitrary combination of two or more thereof. Particularly preferred is graphite.

In accordance with an alternative embodiment of the present invention, silicon carbide is used as source of silicon for preparing the catalyst used in the process of the present invention. Suitable examples therefore are sintered silicon carbide, nitride-bounded silicon carbide, recrystallised silicon carbide, reaction bonded silicon carbide and any arbitrary combination of two or more thereof. Sintered silicon carbide (sSiC) is a self-bonded material containing a sintering aid, such as typically boron, in a content of less than 1% by weight, whereas recrystallized silicon carbide (rSiC) is a high purity silicon carbide having been sintered by a process of evaporation - condensation without any additive. Nitride-bonded silicon carbide (nbSiC), in turn, is prepared by adding fine silicon powder to silicon carbide particles eventually in the presence of a mineral additive and sintering the mixture in a nitrogen furnace. The silicon carbide is bonded by the silicon nitride phase (Si3N₄) formed during nitriding. Reaction bonded silicon carbide (rbSC), which is also denoted as siliconized silicon carbide or siSiC, is a type of silicon carbide, which is manufactured by a chemical reaction between porous carbon or graphite with molten silicon. The silicon reacts with the carbon forming silicon carbide and bonds the silicon carbide particles. Any excess silicon fills the remaining pores in the body and produces a dense silicon carbide-silicium composite. Due to the left-over traces of silicon, reaction bonded silicon carbide is often referred to as siliconized silicon carbide, wherein the process is denoted as reaction bonding, reaction sintering, self-bonding or melt infiltration.

In accordance with yet an alternative embodiment of the present invention, aluminophosphate (ALPO) and/or metalloaluminophosphate (MeAPO) is used as source of silicon for preparing the catalyst used in the process of the present invention. MeAPO's are described in US 4,440,871, US 6,207,872, US 6,540,970 and US 6,303,534. ALPO corresponds to MeAPO, in which the content of Me is 0. If the Me in MeAPO is Si, it is denoted as SAPO. Alternatively, Me ay be a metal being selected from the group consisting of silicon, germanium, magnesium, copper, zinc, iron, cobalt, nickel, manganese, chromium and arbitrary combinations of two or more thereof. Particularly preferred metals are silicon, magnesium and cobalt. In addition, it is preferred that the ALPO and/or MeAPO used in the present invention has the structure AFI (SAPO-5), AEL (SAPO-11), AEI (SAPO-18), ERI (SAPO-17), LEV (SAPO-35), CHA (SAPO-34) or CHA (SAPO-44). More preferably, the ALPO and/or MeAPO used in the present invention the structure CHA or AEI. Furthermore, the MeAPO may be an intergrown phase of two MeAPO's having AEI and CHA framework types, as they are described in US 7,067,095, US 6,953,767 and US 6,334,994.

Furthermore, it is preferable that the support material is substantially free of any strong Bronsted acid sites so as to avoid formation of coke on the catalyst and heavy products. An example of strong acid sites can be found in H-form zeolites with Si/AI framework atomic ratio below 150. However, the presence of acid sites of a moderate strength on the support material can be advantageous in order to improve the dispersion of the metal on the surface, but this is not mandatory. An example of the acid sites of the moderate strength can be found at the gamma alumina support. These type of acid sites due to the presence of a significant amount of co-produced HBr will not impact the selectivity and will not result in high coking rate. Moreover, it is noted that the typical acidic catalysts used in dehydration of alcohols, which are free of metals - such as pure silica alumina, acidic zeolites, or a very pure alumina - are not sufficiently active in dehydrobromination. However, the use of those materials in the reaction still may improve the yield of the thermal process due to better heat transfer and improved hydrodynamics. Nevertheless, the metal free materials are not very efficient catalytically in dehydrobromination, because the co-produced hydrogen bromide is a very strong acid. In contrast thereto, an impurity of a metal on the support at a level of 200 ppm will immediately promote the catalytic reaction.

In accordance with the present invention, in the process a catalyst is used, which comprises a support material and thereon at least 200 ppm of at least one metal being selected from the group consisting of transition metals, noble metals and arbitrary combinations of two or more thereof. Preferably, the support material comprises therein 200 ppm to 20% by weight of one or more transition metals.

The present invention is not particularly limited concerning the kind of transition metal. However, good results are in particular obtained, when the catalyst comprises as transition metal at least one compound being selected from the group consisting of iron, cobalt, nickel, molybdenum, manganese, lead, cupper, chromium, zinc, gallium and arbitrary combinations of two or more thereof.

Moreover, it is preferred that in the process a catalyst is used, which comprises a support material and thereon 200 ppm to 5% by weight of one or more noble metals. The one or more noble metals may be provided on the support material instead of one or more transition metals or in addition to one or more transition metals.

Also concerning the kind of noble metal, the present invention is not particularly limited. However, good results are in particular obtained, when the catalyst comprises as noble metal at least one compound being selected from the group consisting of silver, platinum, palladium, ruthenium, iridium and arbitrary combinations of two or more thereof.

The transition metal and/or noble metal may be introduced onto the support material in any known manner, such as by precipitation, by deposition, by impregnation, by ion exchange, by grafting, by anchoring, by chemical vapor deposition (CVD), by atomic layer epitaxy (ALE) or by encapsulation. Preferably, the transition metal and/or noble metal is introduced onto the support material by impregnation, because this technique allows to load a given porous support material with the transition metal and/or noble metal in a solid-state way (i.e. physical mixture of both component in solid state) or more preferably via wet impregnation (i.e. physical mixture of the support in solid state and the metal component dissolved in a liquid solution).

Incipient wetness impregnation is the most preferred method for the preparation of the catalyst to be used according to the invention, because of its technical simplicity, of its low costs and of the limited amount of waste produced therewith. More specifically, a support is impregnated with a precursor-containing solution and dried at the temperature below 200°C for several hours. The dry product is then further treated through activation treatments, such as calcination and/or reduction, so as to obtain the desired catalyst. The impregnation may be made using the already shaped support material. Preferably, the metal-containing precursorcompound, such as noble metal oxide, transition metal cation or the like, is reduced to the individual metal with hydrogen or an inert hydrocarbon flow at a temperature at least 200°C before the reaction. However, this is not mandatory, because the reduction will take place also under the reaction conditions.

Before loading the transition metals and/or noble metal onto the support material, the support material is preferably shaped, which allows it to be processed to a catalyst having a desired granulometry and good crush strength. This is advantageous, because it allows to limit the pressure drop during the reaction and to prevent that the catalyst breaks down during the reaction into powder-like materials. For this purpose, the support material is preferably shaped, either alone or with addition of a binder. The binder may be selected to be resistant to the temperature and other conditions employed in the processes using the catalyst and the binder may be an inorganic material selected from the same chemical group as the material of the support material, namely silicas, metal silicates, zirconias, borates, aluminas, silica-aluminas, phosphates, for example amorphous aluminophosphates, calcium phosphates, clays, xonotlites, magnesias, metal oxides, such as zirconium dioxide, metals and gels including mixtures of silica and metal oxides. Clays are known to be essentially inert under a wide range of reaction conditions. Suitable clays include commercially available products such as kaolin, kaolinite, montmorillonite, attapulgite, saponite and bentonite. These clays may be used as mined in their natural state or they may also be employed in highly active forms, such as activated by an acid treatment procedure. Clays contribute to strength as a binder enhancing the attrition resistance properties of the catalyst particles, and clays in combination with other binders contributes to the hardness of the particles. Clays also start as small particles and have a higher density, such that when combined with the molecular sieve and binder provide for denser particles, imparting the desirable characteristic of higher density.

It is preferred that for preparing the catalyst, the catalyst particles are combined with the binder material initially by dry-mixing, before the mixture is dispersed in a liquid, preferably water, more preferably together with a plasticizer, so as to yield a paste. Suitable examples of plasticizers as shaping additive are alkylated cellulose derivatives, hydroxyethylcellulose, tylose, ammonium alginate, polyvinyl pyrrolidone, glycerol and polyethylene glycol. These materials will be decomposed during any subsequent heat treatment, e.g. calcination. In addition to enhancing the catalyst strength properties, the binder material allows the molecular sieve crystallite powder to be bound into larger particle sizes suitable for commercial catalytic processes. The formulation of the mixture may be formed into a wide variety of shapes including extrudates, spheres, pills, and the like. The uniformly mixed paste is then subsequently shaped, for example by spray drying to yield microspheres, pelletizing or, preferably or by extrusion. Extrusion may be for example performed in a piston extruder so as to obtain strings, for example cylindrical, which are then dried, again calcined and chopped into pieces of a desired length.

Once the support material is shaped, the transition metal and/or noble metal is loaded onto the support material and the catalyst is present in a substantially dry or dried state, preferably a heat treatment, for example calcination, is performed in order to harden and/to or activate the composition. Preferably, the heat treatment is carried out at a temperature of at least 400°C for a period of 1 to 48 hours. A calcination may be carried out, for example, in a rotary calciner, fluid bed calciner, or a batch oven.

In accordance with a further preferred embodiment of the present invention, the alumina and/or boehmite support material of the catalyst has a silica content of less than 0.4% by weight, has a content of sulfur and sulfur containing compounds of less than 0.5% by weight and has a content of sodium and sodium containing compounds of less than 200 ppm.

Good results are in particular obtained, when the catalyst contains, in sum, alkali earth metals, rare earth metals, compounds containing at least one alkali earth metal and compounds containing at least rare earth metal in an amount of 0.1% by weight to 5% by weight. Preferred alkali earth metals and rare earth metals are calcium, magnesium, yttrium and lanthanum.

Good results are in particular obtained, when the catalyst contains low amounts of or better even no alkali metals. In view of this, it is preferred that the catalyst contains, in sum, alkali metals and compounds containing at least one alkali metal in an amount of less than 1,000 ppm, preferably of less than 200 ppm, more preferably of less than 100 ppm.

In accordance with a further preferred embodiment of the present invention, the catalyst contains, in sum, chlorine in an amount of less than 1,000 ppm, preferably of less than 200 ppm, more preferably of less than 100 ppm, yet more preferably of less than 50 ppm, still more preferably of less than 20 ppm and most preferably of less than 10 ppm.

The present invention is not particularly limited concerning the kind of C₂₋₁₀-bromo-hydrocarbon to be dehydrobrominated to the corresponding C₂₋₁₀-alkene and hydrogen bromide. Thus, the C₂₋₁₀-bromoalkane may be any at least one bromeresidue containing hydrocarbon having from 2 to 10 carbon atoms, such as any C₂₋₁₀-bromoalkane or C₄₋₁₀-bromoalkene. Suitable examples therefore are C₂₋₅-bromoalkanes and particularly C₂₋₄-bromoalkanes. More specific examples for non-cyclic hydrocarbons are monobromoethane, 1,2-dibromoethane, 1-bromopropane, 2-bromopropane, 1,2-dipromopropane, 1,3-dibromopropane, 1-bromo-1-propene, 2-bromo-1-propene, 3-bromo-1-propene, monobromobutane, dibromobutane, monobromopentane, dibromopentane and arbitrary mixtures of two or more of the aforementioned compounds.

In accordance with a particularly preferred embodiment of the present invention, the composition being catalytically reacted contains one or more C₂₋₁₀-bromohydrocarbons and hydrogen, wherein the molar ratio of the one or more C2-10-bromohydrocarbons and the hydrogen is 100:1 to 1:10 and preferably 10:1 to 1:5. The addition of hydrogen to the reaction mixture prevents a potential rebromination of the obtained C2-10-alkene by a reaction of the C2-10-alkene with the co-produced hydrogen bromide. In case of the dehydrobromination of a C2-10-monobromo-alkane, the molar ratio of the one or more C2-10-monobromoalkane and the hydrogen may be comparably low, such as 10:1 to 1:10 and preferably 5:1 to 1:5. However, in the case of the dehydrobromination of a C2-10-dibromoalkane or of a C4-10-monobromoalkene, the molar ratio of i) the one or more C2-10-dibromoalkane or C4-10-monobromoalkene C2-10-monobromoalkane and ii) the hydrogen is higher, such as 5:1 to 1:100 and preferably 2:1 to 1:10.

Because the dehydrobromination reaction is endothermic, it is preferred to add as heat vector an inert component to the reaction mixture so as to bring energy to the reactor. Preferably the weight ratio of the one or more C2-10-bromohydrocarbons and the inert component is preferably 10:1 to 1:10 and preferably 5:1 to 1:5. Suitable examples for the inert component are C1-10-alkanes, steam, nitrogen, argon, methane, carbon dioxide, naphtha and arbitrary combinations of two or more of the aforementioned inert compounds. Good results are in particular obtained, when the inert component is a C₃₋₇-alkane, more preferably a C₄₋₆-alkane and most preferably pentane.

Concerning the type of reactor, in which the dehydrobromination reaction is performed, the present invention is not particularly limited. Preferably, the reaction is performed in a fixed bed reactor, in a radial reactor, in a multi-tubular reactor, in a moving bed reactor or in a fluidized bed reactor. A suitable fluid bed reactor is one of the FCC types used for fluidized-bed catalytic cracking in the oil refinery. A typical moving bed reactor is of the continuous catalytic reforming type. Alternatively, the dehydrobromination reaction may be in a fixed bed reactor configuration using a pair of parallel "swing" reactors. This is possible on account of the high stability of the catalyst to be used in the process of the present invention.

It is proposed in a further development of the idea of the present invention that the reaction is performed at an absolute pressure of 50 kPa to 3 MPa, more preferably of 50 kPa to 1 MPa, yet more preferably of 50 kPa to 500 kPa, still more preferably of 120 kPa to 500 kPa and most preferably of 120 kPa to 400 kPa. Preferably, the pressure is adjusted so that a partial pressure of the formed C₂₋₁₀-alkenes of lower than 2 bars and more preferably of lower than 1 bar is achieved in the effluent of the reactor.

The dehydrobromination reaction is an endothermic reaction and thus requires the input of reaction heat in order to maintain the catalyst activity sufficiently high and in order to shift the thermodynamic equilibrium to sufficiently high reaction levels. In view of this, it is preferred that the reaction is performed at a temperature of 200°C to 500°C, more preferably of 225°C to 450°C and most preferably of 250°C to 400°C.

In the case that the reaction is performed in a fluidized bed reactor using a stationary fluidized bed without catalyst circulation, the reaction temperature is substantially homogeneous throughout the catalyst bed. However, in the case that the reaction is performed in a fluidized bed reactor using a circulating fluidized bed so that the catalyst circulates between a reaction section and a catalyst regeneration section, the temperature in the catalyst bed approaches - depending on the degree of catalyst back mixing - either homogeneous conditions in case of a lot of back mixing or approaches plug flow conditions and hence a decreasing temperature profile will be formed as the reaction proceeds in case that nearly no back mixing occurs. In the case of performing the reaction in a fixed bed reactor or in a moving bed reactor, a decreasing temperature profile will form as the reaction of the proceeds. In this case reaction heat may be introduced by using several catalyst beds in series with inter heating of the reactor effluent from the first bed to higher temperatures and introducing the heated effluent in a second catalyst bed, etc. When the reaction is performed in a fixed bed reactor, a multi-tubular reactor may be used, in which the catalyst is loaded in small-diameter tubes that are installed in the reactor shell. At the shell side, a heating medium may be introduced that provides the required reaction heat by heat-transfer through the wall of the reactor tubes to the catalyst. A radial or spherical reactor may be used to overcome a too import pressure drop.

In accordance with a further preferred embodiment of the present invention, the weight hourly space velocity (WHSV) of the composition being catalytically reacted is 1 to 100 h⁻¹, more preferably 2 to 50 h⁻¹, still more preferably 3 to 20 h⁻¹ and most preferably 4 to 12 h⁻¹. Preferably, the weight hourly space velocity of the composition is measured as the ratio of the weight of the one or more C₂₋₁₀-bromohydro-carbons and the weight of the catalyst, without considering the diluent flow.

In a further development of the idea of the present invention, it is suggested that the catalyst is periodically regenerated by subjecting it in an oxygen containing atmosphere to a temperature of 400 to 650°C and preferably of 420 to 500°C.

Thereby, the carbon on the catalyst is burnt with oxygen. In view of the noble and/or transition metals being contained in the catalyst, it is preferred to limit the exposition of the catalyst to a temperature of at most 650°C and preferably of 500°C. Preferably, the regeneration is performed at about 450°C with a temperature variation across the reactor of at most 50°C. This is typically achieved by limiting the amount of the oxygen in the regeneration loop at the initial stage to at most 1% by mole. After the removal of the main part of the carbon, the oxygen content may be raised to up to 5% by mole. Steam, hydrogen bromide and/or carbon dioxide may be present in the regeneration loop. After the end of the regeneration, it is preferred that all the traces of oxygen are removed from the catalyst, because the presence of oxygen catalyzes the rebromination of the C₂₋₁₀-alkane with hydrogen bromide.

Subsequently, the present invention is further described by means of illustrating, but not restricting examples.

In the figures:
- Fig. 1a and 1b: show the results in terms of conversion of 2-bromopropane and selectivity to propylene of comparative examples 1 and 2 and of examples 1 and 2.
- Fig. 2: shows the results of example 3.
- Fig. 3: shows the results of example 4.
- Fig. 4: shows the results of example 5.
- Fig. 5: shows the results of example 6.

### Examples

### Comparative Example 1

An empty quartz reactor with a 10 mm inner diameter was heated to 450°C in a 3 ml/min nitrogen flow at atmospheric pressure followed by stopping the nitrogen flow and switching to 3 Nl/h of hydrogen for 1 h. Then, the reactor was cooled down from 450°C in hydrogen flow to the reaction temperature of 350°C. Thereafter, 4.8 ml/h (6.3 g/h) of 2-bromopropane and of 15.64 ml/min hydrogen were fed to the reactor at atmospheric pressure.

The reaction was performed during 9 hours with the on-line GC analysis.

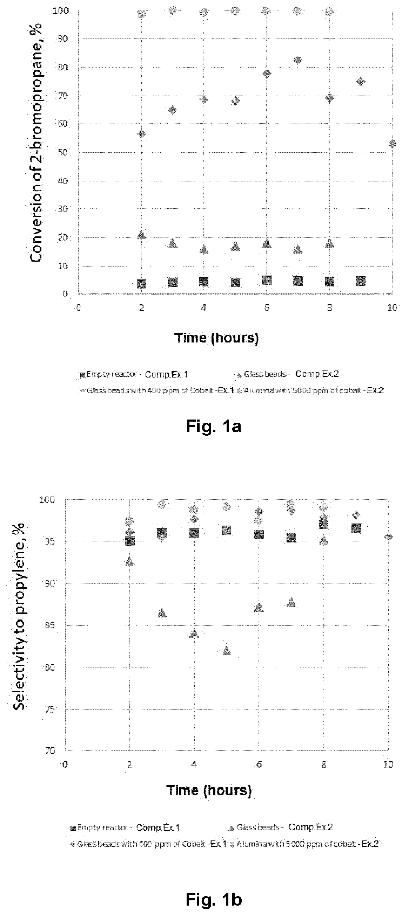

The results are shown in figure 1a in terms of conversion in % (on the abscissa; calculated on carbon basis, coke free basis) of 2-bromopropane in dependency of the reaction time in hours (ordinate) and in figure 1b in terms of selectivity in % (on the abscissa; calculated on carbon basis, coke free basis) to propylene in dependency of the reaction time in hours (ordinate) of comparative examples 1 and 2 and of examples 1 and 2.

These results show that the dehydrobromination does occur in the absence of any catalyst thermally, but only in an insignificant extent so that it is not suitable to be used industrially. Also, the selectivity of the reaction to propylene is low and hardly controllable.

### Comparative Example 2

10 g of beads with a diameter of 1 mm and made from metal free silica were loaded in a quartz reactor of 10 mm in inner diameter. The reactor was heated to 450°C in 3 ml/min nitrogen flow at atmospheric pressure followed by stopping the nitrogen flow and switching to 3 NI/h of hydrogen for 1 h. Then, the reactor was cooled down from 450°C in hydrogen flow to the reaction temperature of 350°C. Then, the nitrogen was stopped and 4.8 ml/h (6.3 g/h) of 2-bromopropane and of 15.64 ml/min hydrogen were fed to the reactor at atmospheric pressure.

The reaction was performed during 8 hours with the on-line GC analysis

The results are shown in figure 1a in terms of conversion in % (on the abscissa; calculated on carbon basis, coke free basis) of 2-bromopropane in dependency of the reaction time in hours (ordinate) and in figure 1b in terms of selectivity in % (on the abscissa; calculated on carbon basis, coke free basis) to propylene in dependency of the reaction time in hours (ordinate) of comparative examples 1 and 2 and of examples 1 and 2.

These results show that the addition of an inert support (beads with a diameter of 1 mm and made from metal free silica) allows to increase the thermal conversion of 2-bromopropane in comparison to comparative example 1 thanks to a better heat transfer and to an improved hydrodynamics. However, the performance remains modest and insufficient for a commercial unit.

### Example 1

10 g of beads with a diameter of 1 mm and made from metal free silica as support material were immersed in cobalt nitrates hexahydrate aqueous solution so as to introduce 400 ppm of cobalt as transition metal on the support material. The water was slowly evaporated. The so treated glass beads were loaded in a quartz reactor with an inner diameter of 10 mm and heated to 450°C in 3 ml/min nitrogen flow at atmospheric pressure followed by stopping the nitrogen flow and switching to 3 NI/h of hydrogen for 1 h. Then, the reactor was cooled down from 450°C in hydrogen flow to the reaction temperature of 350°C. Thereafter, 4.8 ml/h (6.3 g/h) of 2-bromopropane and of 15.64 ml/min hydrogen were fed to the reactor at atmospheric pressure.

The reaction was performed during 10 hours with the on-line GC analysis.

The results are shown in figure 1a in terms of conversion in % (on the abscissa; calculated on carbon basis, coke free basis) of 2-bromopropane in dependency of the reaction time in hours (ordinate) and in figure 1b in terms of selectivity in % (on the abscissa; calculated on carbon basis, coke free basis) to propylene in dependency of the reaction time in hours (ordinate) of comparative examples 1 and 2 and of examples 1 and 2.

These results show that the addition of a catalyst in accordance with the present invention (here a catalyst of silica support containing 400 ppm cobalt as transition metal) drastically increases the conversion rate of 2-bromopropane and the selectivity.

### Example 2

A powder a gamma-alumina as support material being prepared by the aluminium alkoxide route having a specific surface area of 180 m²/g, having a content of sodium compounds of less than 100 ppm and having a silica content of less than 100 ppm, was incipiency wetness impregnated with an aqueous solution of cobalt nitrates hexahydrate so as to obtain 0.5% by weight (i.e. 5,000 ppm) by of cobalt on the support material. The material was dried for 24 h at 100°C and was then subjected to calcination at 550°C for 5 h in an air flow (ramp = 5 °C and hold = 5 h). The so obtained material was pelletized and then crushed and seized to a 35 to 45 mesh fraction. Two grams of the fraction of the catalyst were loaded in a fixed-bed tubular reactor with an inner diameter of 10 mm. The loaded catalyst was heated up in 3 Nl/h nitrogen flow to 450°C at atmospheric pressure followed by stopping the nitrogen flow and switching to 3 NI/h of hydrogen for 1 h. Then, the reactor was cooled down from 450°C in hydrogen flow to 350°C. Thereafter, 4.8 ml/h (6.3 g/h) of 2-bromopropane and of 15.64 ml/min hydrogen were fed to the reactor at atmospheric pressure.

The reaction was performed during 8 hours with the on-line GC analysis.

The results are shown in figure 1a in terms of conversion in % (on the abscissa; calculated on carbon basis, coke free basis) of 2-bromopropane in dependency of the reaction time in hours (ordinate) and in figure 1b in terms of selectivity in % (on the abscissa; calculated on carbon basis, coke free basis) to propylene in dependency of the reaction time in hours (ordinate) of comparative examples 1 and 2 and of examples 1 and 2.

These results show that the addition of a catalyst in accordance with the present invention (here a catalyst of silica support containing 5,000 cobalt as transition metal) drastically increases the conversion rate of 2-bromopropane and the selectivity.

### Example 3

Two grams of gamma-alumina as support material having a specific surface area of 180 m²/g, having a content of sodium compounds of less than 100 ppm and having a silica content of less than 100 ppm were incipiency wetness impregnated with a solution of iron(III)nitrates so as to obtain about 5% by weight of iron on the support material. The material was dried for 24 h at 100°C and subjected to calcination at 550°C for 5 h in an air flow (ramp = 5 °C and hold = 5 h). The so obtained material was pelletized and then crushed and seized to a 35 to 45 mesh fraction.

Two grams of the so obtained fraction of the catalyst containing 5wt% of iron on alumina was loaded in a fixed-bed tubular quartz reactor with an inner diameter of 10 mm. The dead volume above the catalyst in the reactor was filled with spherical silica beads. The loaded catalyst was heated up in 3 NI/h nitrogen flow to 450°C at atmospheric pressure followed by stopping the nitrogen flow and switching to 3 NI/h of hydrogen for 1 h. Then, the reactor was cooled down from 450°C in hydrogen flow to the reaction temperature, the hydrogen flow was increased to 48.6 ml/min and the gaseous 2-bromopropane (16 g/h) was fed into the reactor.

The conversion and selectivity to propylene (the catalyst performance) in the catalytic dehydrobromination of 2-bromopropane on the prepared above catalyst were measure for 50 hours on-stream at temperature 300°C, WHSV (2-bromopropane) 8 h⁻¹, hydrogen/2-bromopropane molar ratio of 1. Then the temperature was increased to 350°C and the performance was measured for 25 additional hours on-stream. Afterwards, the hydrogen flow was replaced by the same flow on nitrogen and the test was continued for 25 additional hours on-stream keeping the rest of the parameters the same.

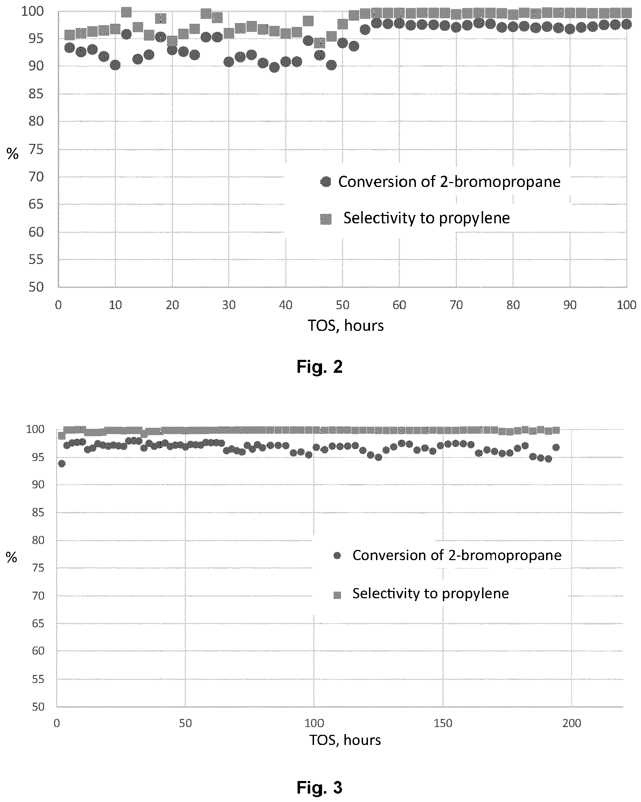

The results are shown in figure 2. The abscissa shows the time on stream in hours and the ordinate shows the % of the conversion (circle data points) and of the selectivity (square data points). Again, the conversion of 2-bromopropane was calculated on carbon basis, coke free basis and the selectivity was calculated on carbon basis, coke free basis.

The results show that the used catalyst assures a stable conversion of bromopropane and a very high selectivity to propylene of above 99%.

### Example 4

Two grams of the gamma-alumina as support material having a specific surface area of 180 m²/g, having a content of sodium compounds of less than 100 ppm and having a silica content of less than 100 ppm were incipiency wetness impregnated with a solution of cobalt nitrates so as to obtain about 5% by weight of cobalt on the support material. The material was dried for 24 h at 100°C and subjected to calcination at 550°C for 5 h in an air flow (ramp = 5 °C and hold = 5 h). The obtained material was pelletized and then crushed and seized to a 35 to 45 mesh fraction.

Two grams of the so obtained fraction of the catalyst containing 5wt% of Co on alumina was loaded in a fixed-bed tubular quartz reactor with an inner diameter of 10 mm. The dead volume above the catalyst in the reactor was filled with spherical silica beads. The loaded catalyst was heated up in 3 NI/h nitrogen flow to 450°C at atmospheric pressure followed by stopping the nitrogen flow and switching to 3 Nl/h of hydrogen for 1 h. Then, the reactor was cooled down from 450°C in hydrogen flow to the reaction temperature, the hydrogen flow was increase to 48.6 ml/min and the gaseous 2-bromopropane (16 g/h) was sent into the reactor.

The conversion and selectivity to propylene (the catalyst performance) in the catalytic dehydrobromination of 2-bromopropane on the prepared above catalyst were measure for about 200 hours on-stream at temperature 350°C, WHSV (2-bromopropane) 8 h⁻¹, hydrogen/2-bromopropane molar ratio of 1.

The results are shown in figure 3. The abscissa shows the time on stream in hours and the ordinate shows the % of the conversion (circle data points) and of the selectivity (square data points). Again, the conversion of 2-bromopropane was calculated on carbon basis, coke free basis and the selectivity was calculated on carbon basis, coke free basis.

The results show that the used catalyst assures a stable conversion of bromopropane and a very high selectivity to propylene of above 99%.

### Example 5

Example 4 was repeated except that the catalyst was additionally impregnated with 2,000 ppm by weight of platinum added as tetraamminplatin(II)-nitrate (Pt(NH₃)₄(NO₃)₂) and with 0.5% by weight of calcium as calcium nitrate (Ca(NO₃)₂) followed by the same drying and calcination procedures as in example 4.

The same catalyst loading (2g of catalyst, 35 to 45 mesh) and the same activation procedure was used as in example 4.

In order to assess the impact of the 1,2-dibromopropane impurities in bromopropane, the experiment was started with a pure 2-bromopropane (WHSV-4h⁻¹) feedstock, with H₂-feed molar ratio of 0.2, with a nitrogen flow of 26 ml/min at a temperature of 350°C and at atmospheric pressure (TOS: 1 to 6 h). Then, the feedstock was switched to a mixture of 98% by weight of 2-bromopropane and of 2% by weight of 1,2- dibromopropane without changing hydrogen and nitrogen flow to the reactor at atmospheric pressure for 30 hours (TOS: 6 to 36 hours).

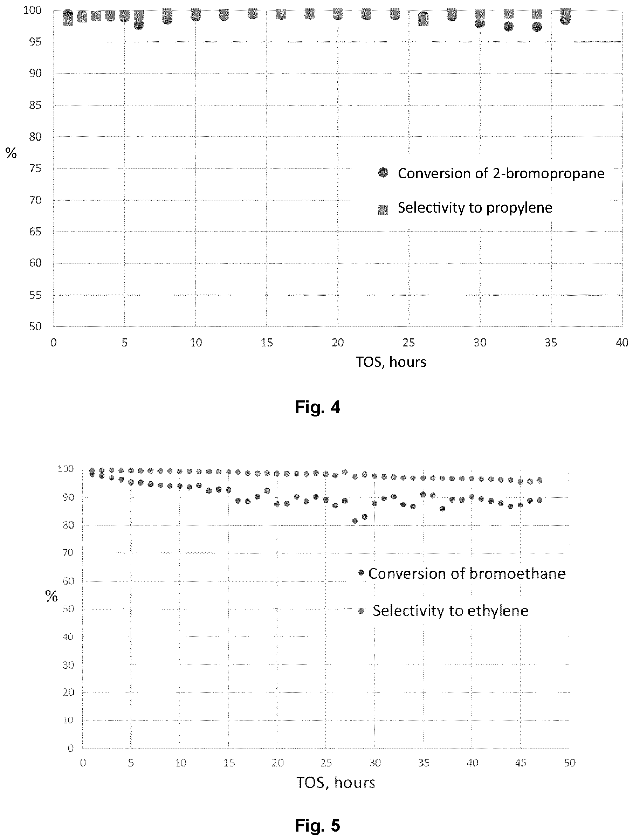

The results are shown in figure 4. The abscissa shows the time on stream in hours and the ordinate shows the % of the conversion (circle data points) and of the selectivity (square data points). Again, the conversion was calculated on carbon basis, coke free basis and the selectivity was calculated on carbon basis, coke free basis.

The results show no changes in the performance after switching to a mixture of 2-bromopropane with 2% by weight of 1,2- di-bromopropane. That shows that the catalysts according to the invention is also efficient in processing of di-bromo-alkanes.

### Example 6

In this example the sample of the catalyst prepared in the example 4 was tested concerning its bromoethane conversion.

Two grams of the 35 to 45 mesh fraction of the catalyst made of 5% by weight of cobalt on alumina as prepared in example 4 was loaded in a fixed-bed tubular quartz reactor with an inner diameter of 10 mm. The dead volume above the catalyst in the reactor was filled with spherical silica beads. The loaded catalyst was heated up in 3 NI/h nitrogen flow to 450°C at atmospheric pressure followed by stopping the nitrogen flow and switching to 3 NI/h of hydrogen for 1 h. Then, the reactor was cooled down from 450°C to 375°C in hydrogen flow to the reaction temperature, the hydrogen flow was increased to 27.4 ml/min and bromoethane was sent in a flow of 8 g/h into the reactor under atmospheric pressure. The test was performed during 47 hours on-stream (TOS, h).

The results are shown in figure 5. The abscissa shows the time on stream in hours and the ordinate shows the % of the conversion (circle data points) and of the selectivity (square data points). Again, the conversion was calculated on carbon basis, coke free basis and the selectivity was calculated on carbon basis, coke free basis.

The results show that the catalyst used in example 5 is also very active and selective in bromoethane dehydrobromination.

## Claims

1. A process for catalytically reacting a composition containing one or more C₂₋₁₀-bromohydrocarbons to the corresponding C₂₋₁₀-alkenes, wherein the reaction is performed in the presence of a catalyst comprising a support material and thereon at least 200 ppm of at least one metal being selected from the group consisting of transition metals, noble metals and arbitrary combinations of two or more thereof.

2. The process in accordance with claim 1, wherein the content of the support material of the catalyst is at least 75% by weight.

3. The process in accordance with claim 1 or 2, wherein the support material has a specific surface area, measured by nitrogen adsorption, of at least 5 m²/g more, preferably of at least 20 m²/g and more preferably of at least 50 m²/g.

4. The process in accordance with any of the preceding claims, wherein the support material is selected from the group consisting of aluminas, silicas, silica gels, silica-aluminas, zeolites, zirconias, titanias, niobias, silicon carbides, carbons, aluminophosphates molecular sieves, metalloaluminophosphate molecular sieves, amorphous aluminophosphates and arbitrary combinations of two or more thereof.

5. The process in accordance with with any of the preceding claims, wherein the support material of the catalyst is gamma-alumina and/or boehmite.

6. The process in accordance with with any of the preceding claims, wherein the catalyst comprises 200 ppm to 20% by weight of one or more transition metals.

7. The process in accordance with with any of the preceding claims, wherein the catalyst as transition metal at least one compound being selected from the group consisting of iron, cobalt, nickel, molybdenum, manganese, lead, cupper, chromium, zinc, gallium and arbitrary combinations of two or more thereof.

8. The process in accordance with with any of the preceding claims, wherein the catalyst comprises 200 ppm to 5% by weight of one or more noble metals.

9. The process in accordance with with any of the preceding claims, wherein the catalyst as noble metal at least one compound being selected from the group consisting of silver, platinum, palladium, ruthenium, iridium and arbitrary combinations of two or more thereof.

10. The process in accordance with any of the preceding claims, wherein the alumina or gamma-alumina and/or boehmite support material of the catalyst has a silica content of less than 0.4% by weight, has a content of sulfur and sulfur containing compounds of less than 0.5% by weight and has a content of sodium and sodium containing compounds of less than 200 ppm.

11. The process in accordance with any of the preceding claims, wherein the catalyst contains, in sum, alkali metals and compounds containing at least one alkali metal in an amount of less than 1,000 ppm, preferably of less than 200 ppm, more preferably of less than 100 ppm, yet more preferably of less than 50 ppm, still more preferably of less than 20 ppm and most preferably of less than 10 ppm.

12. The process in accordance with any of the preceding claims, wherein the catalyst contains, in sum, chlorine in an amount of less than 1,000 ppm, preferably of less than 200 ppm, more preferably of less than 100 ppm, yet more preferably of less than 50 ppm, still more preferably of less than 20 ppm and most preferably of less than 10 ppm.

13. The process in accordance with any of the preceding claims, wherein a C₂₋₁₀-bromoalkane, C₂₋₅-bromoalkane, more preferably a C₂₋₄-bromoalkane and most preferably bromopropane is catalytically reacted to the corresponding alkenes.

14. The process in accordance with any of the preceding claims, wherein the composition being catalytically reacted contains one or more inert compounds being preferably selected from the group consisting of C₁₋₁₀-alkanes, steam, nitrogen, argon, methane, carbon dioxide, naphtha and arbitrary combinations of two or more of the aforementioned inert compounds.

15. The process in accordance with any of the preceding claims, wherein the reaction is performed in a fixed bed reactor, in a radial reactor, in a multitubular reactor, in a moving bed reactor or in a fluidized bed reactor, wherein preferably the reaction is performed at an absolute pressure of 50 kPa to 3 MPa, preferably of 50 kPa to 1 MPa, more preferably of 50 kPa to 500 kPa, still more preferably of 120 kPa to 500 kPa and most preferably of 120 kPa to 400 kPa, wherein preferably the reaction is performed at a temperature of 200°C to 500°C, preferably of 225°C to 450°C and more preferably of 250°C to 400°C, and wherein preferably the weight hourly space velocity of the composition being catalytically reacted is 1 to 100 h⁻¹, preferably 2 to 50 h⁻¹, more preferably 3 to 20 h⁻¹ and most preferably 4 to 12 h⁻¹.
